# DEMANDE DE BREVET EUROPEEN

(11) **EP 2 036 590 A2**
(43) Date de publication de la demande: **18.03.2009**
(21) Numéro de dépôt: 08164231.6
(22) Date de dépôt: 12.09.2008
(51) Int. Cl.: A61Q 5/00, A61Q 5/02, A61Q 5/12, A61K 8/73, A61K 8/91, A61K 8/72

(54) **Compositions cosmétiques contenant un copolymère cationique, une cyclodextrine et un tensioactif et leurs utilisations.**

(30) Priorité: 14.09.2007 FR 0757581
(71) Demandeur: L'Oréal, 75008 Paris (FR)
(72) Inventeur: Biganska, Olga, 92600 Asnières sur Seine (FR)
(74) Mandataire: Le Blainvaux Bellegarde, Françoise

(57) **Abrégé**

La présente invention a pour objet des compositions cosmétiques comprenant dans un milieu aqueux :
(i)- un ou plusieurs polymères cationiques qui sont produits par polymérisation d'un mélange de monomères comprenant :
a) un ou plusieurs monomères vinyliques substitués par un ou plusieurs groupes amino,
b) un ou plusieurs monomères vinyliques non ioniques hydrophobes, choisis parmi ceux de formules (I) et (II) :

(I) CH₂=C(X)Z,

(II) CH₂=CH-OC(O)R;

dans lesquelles formules (I) et (II) :
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et-(CH₂)ₓSiR₃ ;
x représente un nombre entier allant de 1 à 6 ;
chaque R représente indépendamment un groupe alkyle en C₁-C₃₀ ; chaque R¹ représente indépendamment un groupe alkyle en C₁-C₃₀, un groupe alkyle en C₂-C₃₀ hydroxylé, ou un groupe alkyle en C₁-C₃₀ halogéné et
c) un ou plusieurs monomères vinyliques associatifs,
e) un ou plusieurs monomères vinyliques non ioniques hydroxylés,

(ii)- une ou plusieurs cyclodextrines ou l'un de ses dérivés, et
(iii)-un ou plusieurs tensioactifs.

Elle a aussi pour objet des compositions comprenant en outre un ou plusieurs agents de conditionnement. L'invention a également pour objet l'utilisation du polymère cationique (i) en tant qu'agent de suspension de la cyclodextrine .

Les compositions selon l'invention sont utilisées en particulier comme produits rincés notamment pour le lavage et/ou le conditionnement des matières kératiniques.

## Description

La présente invention a pour objet des compositions comprenant dans un milieu aqueux physiologiquement acceptable au moins une cyclodextrine, au moins un tensioactif et au moins un copolymère cationique particulier. Elle a aussi pour objet des compositions comprenant en outre au moins un agent de conditionnement. L'invention a également pour objet l'utilisation du copolymère cationique particulier en tant qu'agent de suspension du complexe formé de la cyclodextrine et du tensioactif.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.

Compte tenu du caractère insoluble de certains agents de conditionnement tels que par exemple les silicones ou les huiles, on cherche à maintenir les agents de conditionnement en dispersion régulière dans le milieu sans cependant faire chuter la viscosité et les propriétés détergentes et moussantes des compositions. Les agents de conditionnement notamment les silicones ou les huiles doivent également être véhiculés sur les matières kératiniques traitées en vue de leur conférer, suivant l'application, des propriétés de douceur, de brillance et de démêlage.

On sait également que les produits en particulier cosmétiques ayant un aspect ou un effet irisé, moiré ou métallisé, sont largement appréciés par les consommateurs pour leur aspect esthétique et pour l'apparence de richesse du produit. Les agents qui apportent cet effet sont des agents de nacrage ou agent nacrants comprenant généralement des cristaux qui restent dispersés dans les compositions et qui réfléchissent la lumière.
Les dérivés d'esters à longue chaîne sont largement utilisés pour nacrer les compositions notamment cosmétiques. Cependant, ces dérivés peuvent présenter des problèmes de cristallisation qui entraînent une évolution de la viscosité des compositions au cours du temps.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

Il a été constaté que ces agents nacrants, du fait de leur faible densité, présentaient souvent l'inconvénient de remonter à la surface du shampooing et d'y former une couche inesthétique pour le consommateur.

De plus ces composés à chaînes grasses présentent dans certains cas l'inconvénient d'apporter un toucher chargé aux cheveux, un manque de légèreté, de volume de la chevelure .

Il existe donc toujours un besoin pour de nouveaux agents nacrants qui ne présentent pas les inconvénient cités ci-dessus et qui permettent également l'utilisation d'agents de conditionnement insolubles et en particulier les silicones.
Pour nacrer les compositions notamment de shampooing, on a déjà utiliser des cyclodextrines, voir notamment le document EP1499280. Cependant les compositions ne sont pas suffisamment stables au stockage en particulier à température élevés (environ 45°C) et le nacrage peut disparaître au cours du temps.

Pour épaissir et stabiliser les compositions cosmétiques contenant des agents conditionneurs insolubles, des agents de stabilisation tels que les polymères acryliques réticulés du type Carbopol 980 sont fréquemment utilisés. Néanmoins, ces agents de stabilisation présentent l'inconvénient de diminuer les performances cosmétiques des shampooings, notamment en rendant les cheveux plus chargés et plus rêches. De plus les compositions ne sont pas encore suffisamment stables au stockage en particulier à températures élevées (environ 45°C). Le nacrage disparaît au cours du temps.

EP1779840 divulgue des compositions comprenant un tensioactif, une cyclodextrine et un polymère acrylique. Les polymères acryliques sont différents des copolymères cationiques selon l'invention.

La demanderesse a découvert qu'il était possible de formuler des compositions notamment cosmétique pour le traitement des matières kératiniques, en particulier des shampooings présentant un aspect nacré tout en ayant les propriétés esthétiques et cosmétiques recherchées, en utilisant dans ces compositions au moins une cyclodextrine, au moins un tensioactif et au moins un polymère cationique particulier.

L'invention a pour objet des compositions notamment cosmétiques comprenant dans un milieu aqueux notamment physiologiquement acceptable et en particulier cosmétiquement acceptable :
(i)- un ou plusieurs polymères cationiques qui sont produits par polymérisation d'un mélange de monomères comprenant :
   a) un ou plusieurs monomères vinyliques substitués par un ou plusieurs groupes amino,
   b) un ou plusieurs monomères vinyliques non ioniques hydrophobes,
   choisis parmi ceux de formules (I) et (II):

   (I) CH₂=C(X)Z,

   (II) CH₂=CH-OC(O)R;

   dans lesquelles formules (I) et (II):
   X représente H ou un groupe méthyle;
   Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃;
   x représente un nombre entier allant de 1 à 6;
   chaque R représente indépendamment un groupe alkyle en C₁-C₃₀; chaque R¹ représente indépendamment un groupe alkyle en C₁-C₃₀, un groupe alkyle en C₂-C₃₀ hydroxylé, ou un groupe alkyle en C₁-C₃₀ halogéné et
      c) un ou plusieurs monomères vinyliques associatifs,
      e) un ou plusieurs monomères vinyliques non ioniques hydroxylés,
(ii)- une ou plusieurs cyclodextrines ou l'un de ses dérivés, et
(iii)- un ou plusieurs tensioactifs.

Les compositions selon l'invention présentent l'avantage de s'étaler facilement et de se répartir de façon régulière le long des fibres kératiniques.

La présence de ce copolymère ne diminue pas les propriétés cosmétiques des compositions et peut même les améliorer, notamment en ce qui concerne la souplesse, le lissage, la brillance, le volume et/ou la discipline.

Les compositions présentent une très bonne homogénéité et une bonne stabilité du nacrage, ainsi qu'une viscosité satisfaisante pour l'application sur les matières kératiniques. Il n'y a pas de déphasage (séparation de phase) ni de disparition du nacrage au cours du temps.

En particulier, il ne se produit aucun relargage ou épaississement incontrôlé de la composition au cours du temps. Les compositions présentent enfin une texture non filante et fondante. La mousse se rince facilement.

L'invention a encore pour objet l'utilisation d'un ou plusieurs polymères cationiques (i) en tant qu'agent de mise en suspension du complexe insoluble formé par la cyclodextrine et un tensioactif dans une composition cosmétique en particulier de lavage et/ou de conditionnement comprenant au moins un milieu aqueux physiologiquement acceptable comprennant une ou plusieurs cyclodextrines et un ou plusieurs tensioactifs.

Un autre objet de l'invention est constitué par le procédé de lavage et/ou de conditionnement mettant en oeuvre de telles compositions.

Un autre objet de l'invention concerne l'utilisation d'un ou plusieurs polymères cationiques (i) tel que décrit ci-dessous dans, ou pour la fabrication d'une composition cosmétique comprenant une ou plusieurs cyclodextrines et un ou plusieurs tensioactifs.

D'autres objets de l'invention apparaîtront à la lecture de la description et des exemples qui suivent.
Selon la présente invention, par matières kératiniques, on comprend les cheveux, les cils, les sourcils, la peau, les ongles, les muqueuses ou le cuir chevelu et plus particulièrement les cheveux.
Par le terme "agent de nacrage" ou "agent nacrant", on entend un agent produisant un aspect ou un effet nacré, irisé, moiré ou métallisé.

Par monomère hydrophobe, on entend au sens de la présente invention, un monomère qui présente une solubilité dans l'eau inférieure à 10g pour 100 mL d'eau à une température de 20°C.

L'une des caractéristiques essentielles de l'invention est la présence d'un polymère cationique qui est obtenu par polymérisation d'un mélange de monomères comprenant a) un ou plusieurs monomères vinyliques substitués par un ou plusieurs groupes amines, b) un ou plusieurs monomères vinyliques non ioniques hydrophobes, c) un ou plusieurs monomères vinyliques associatifs et e) un ou plusieurs monomères vinyliques non ioniques hydroxylés . De préférence, les monomères constituant le copolymère cationique comprennent en outre un ou plusieurs monomères tensioactifs vinyliques semi-hydrophobes d). Les monomères a) à e) sont différents les uns des autres.

De préférence, le polymère cationique (i) est un polymère épaississant.

Au sens de la présente invention, on entend par polymère épaississant, un polymère qui, introduit à 1 % en poids dans une solution aqueuse ou hydroalcoolique à 30 % en poids d'éthanol, et à pH 7, permet d'atteindre une viscosité d'au moins 100 cps à 25 °C, et à un taux de cisaillement de 1 s⁻¹. Cette viscosité peut être mesurée à l'aide d'un viscosimètre à géométrie cône-plan, par exemple, un rhéomètre Haake RS 600. De préférence, ces polymères permettent d'augmenter la viscosité des compositions dans lesquelles ils se trouvent, d'au moins 50 cps à 25 °C et à 1 s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant un ou plusieurs groupes R₀CH=C(R₀)-, ou chaque R₀ est indépendamment H, un alkyle en C₁-C₃₀, -C(O)OH ou C(O)OR₀', -OC(O)OR₀', -C(O)NHR₀', C(O)NR₀' R₀" ,R₀' et R₀", identiques ou différents, étant un groupe alkyle en C₁-C₃₀.
Ainsi, par exemple, au sens de la présente invention, les (méth)acrylates et les (méth)acrylamides sont des monomères vinyliques.
Comme expliqué précédemment, le mélange de monomères permettant la préparation du polymère cationique (i) utilisé dans la composition selon l'invention comprend un ou plusieurs monomères vinyliques substitués par un ou plusieurs groupes amino.

Les monomères vinyliques substitués par un ou plusieurs groupes amino utilisables pour la préparation du polymère cationique utilisé selon l'invention sont des monomères à insaturation éthylénique, basiques et polymérisables. Les groupes amines peuvent dériver de groupes alkyles mono, di- ou polyaminés, ou bien de groupes hétéroaromatiques comprenant un atome d'azote. Les groupes amines peuvent être des amines primaires, secondaires ou tertiaires. Ces monomères peuvent être utilisés sous la forme d'amine ou sous la forme de sel.
De préférence, le ou les monomères vinyliques substitués par un ou plusieurs groupes amines sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides à groupement(s) hétérocyclique(s) contenant un atome d'azote,
- les (méth)acrylates à groupement(s) hétérocyclique(s) contenant un atome d'azote,
- les hétérocycles azotés à groupement(s) vinyle(s),
et leurs mélanges.
A titre de monomères vinyliques substitués par un ou plusieurs groupes amino préférés, on peut citer :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.
Lorsque les monomères sont sous forme de sels, il peut s'agir de sels minéraux, tels que les sels hydrochlorure, sulfate et phosphate ; ou bien de sels d'acides organiques, tels que les sels acétate, maléate et fumarate.
Des monomères vinyliques substitués par un ou plusieurs groupe(s) amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.
Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.

Comme expliqué précédemment, le mélange de monomères permettant la préparation du polymère cationique utilisé (i) selon l'invention comprend également un ou plusieurs monomères vinyliques non ioniques hydrophobes b).
Le ou les monomères vinyliques non ioniques hydrophobes utilisables pour la préparation du polymère cationique utilisé selon l'invention sont généralement choisis parmi les composés de formules (I) et (II):

(I) CH₂=C(X)Z,

(II) CH₂=CH-OC(O)R;

où, dans chacune des formules (I) et (II);
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃;
x représente un nombre entier allant de 1 à 6;
chaque R représente indépendamment un groupe alkyle en C₁-C₃₀;
chaque R¹ représente indépendamment un groupe alkyle en C₁-C₃₀, un groupe alkyle en C₂-C₃₀ hydroxylé, ou un groupe alkyle en C₁-C₃₀ halogéné.
On peut citer en particulier les (méth)acrylates d'alkyle en C₁-C₃₀; les (alkyl en C₁-C₃₀)(méth)acrylamides; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.
De préférence, le ou les monomères vinyliques non ioniques hydrophobes sont choisis parmi les acrylates d'alkyle en C₁-C₃₀, les méthacrylates d'alkyle en C₁-C₃₀, et leurs mélanges, tels que l'acrylate d'éthyle, le méthacrylate de méthyle, le méthacrylate de 3,3,5-triméthylcyclohexyle.
Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé selon l'invention sont généralement choisis parmi des composés ayant une extrémité (i)' à insaturation(s) éthylénique(s) pour la polymérisation par addition avec d'autres monomères du système ; une portion centrale (ii)' polyoxyalkylène pour conférer des propriétés hydrophiles sélectives aux polymères, et une extrémité (iii)' hydrophobe pour conférer des propriétés hydrophobes sélectives aux polymères.
L'extrémité (i)' à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i)' du monomère associatif peut être dérivée d'un allyl éther ou d'un vinyl éther ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.
La portion centrale (ii)' du ou des monomères vinyliques associatifs est de préférence un segment polyoxyalkylène comprenant 5 à 250, de préférence encore 10 à 120, et mieux 15 à 60 unités oxydes d'alkylène en C₂-C₇. Des portions centrales (ii)' préférées sont les segments polyoxyéthylène, polyoxypropylène, et polyoxybutylène comprenant 5 à 150, de préférence 10 à 100, et mieux 15 à 60 unités oxydes d'éthylène, de propylène ou de butylène, et des séquences aléatoires ou non aléatoires d'unités oxydes d'éthylène, oxydes de propylène ou oxydes de butylène. De préférence, les portions centrales sont des segments polyoxyéthylène.
L'extrémité (iii)' hydrophobe du ou des monomères associatifs est de préférence un fragment hydrocarboné appartenant à l'une des classes hydrocarbonées suivantes : un alkyle linéaire en C₈-C₄₀, un alkyle en C₂-C₄₀ substitué par un groupe aryle, un phényle substitué par un groupe alkyle en C₂-C₄₀, un alkyle ramifié en C₈-C₄₀, un groupe alicyclique en C₈-C₄₀, et un ester complexe en C₈-C₈₀.
Par ester complexe, on entend au sens de la présente invention tout ester différent d'un ester simple.
Par ester simple, on entend au sens de la présente invention tout ester d'alcool aliphatique saturé en C₁-C₃₀, linéaire ou ramifié, et non substitué.

Des exemples d'extrémités (iii)' hydrophobes du ou des monomères associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).
Des exemples de groupes alkyles linéaires ou ramifiés ayant 8 à 40 atomes de carbone et dérivés d'une source naturelle sont notamment les groupes alkyles dérivés de l'huile d'arachide hydrogénée, d'huile de soja et d'huile de canola (à prédominance C₁₈), l'huile de suif hydrogénée en C₁₆-C₁₈; et les terpénols hydrogénés en C₁₀-C₃₀, tels que le géraniol hydrogéné (en C₁₀ ramifié), le farnesol hydrogéné (C₁₅ ramifié), le phytol hydrogéné (C₂₀ ramifié).
Des exemples de phényles substitués par un alkyle en C₂-C₄₀ sont l'octylphényle, le nonylphényle, le décylphényle, le dodécylphényle, l'hexadécylphényle, l'octadécylphényle, l'isooctylphényle et le sec-butylphényle.
Des groupes alicycliques en C₈-C₄₀ peuvent être par exemple les groupes dérivés des stérols d'origine animale, tels que le cholestérol, le lanostérol, le 7-déhydrocholestérol ; ou bien les dérivés d'origine végétales, tels que le phytostérol, le stigmastérol, le campestérol ; ou bien les dérivés issus de microorganismes, tels que l'ergostérol, le mycrostérol. D'autres alicycliques en C₈-C₄₀ utilisables dans la présente invention sont par exemple le cyclooctyle, le cyclododécyle, l'adamantyle, le décahydronaphthyle, et les groupes dérivés de composés alicycliques en C₈-C₄₀ naturels tels que le pinène, le rétinol hydrogéné, le camphre et l'alcool d'isobornyle.
Les groupes alkyles en C₂-C₄₀ substitués par un groupe aryle peuvent être par exemple le 2-phényléthyle, le 2,4-diphénybutyle, le 2,4,6-triphénylhexyle, le 4-phénylbutyle, le 2-méthyl-2-phényléthyle, le 2,4,6-tri(1'phényléthyl)phényle.
A titre d'esters complexes en C₈-C₄₀ utilisables comme extrémité (iii), on peut citer l'huile de ricin hydrogénée (principalement le triglycéride de l'acide 12-hydroxystéarique; les 1,2-diacyl glycérols tels que le 1,2-distéaryl glycérol, le 1,2-dipalmityl glycérol, le 1,2-dimyristyl glycérol ; les di-, tri- ou poly-esters de sucres tel que le 3,4,6-tristéaryl glucose, le 2,3-dilauryle fructose ; et les esters de sorbitane tels que ceux divulgués dans le brevet US 4,600,761.
Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.
De préférence, le ou les monomères vinyliques associatifs c) utilisables selon l'invention sont choisis parmi les composés de formule (III): 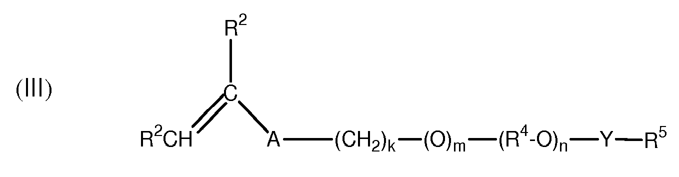 Dans laquelle
chaque R² est indépendamment H, un groupe méthyle, un groupe -C(O)OH, ou un groupe -C(O)OR³;
R³ est un alkyle en C₁-C₃₀;
A est un groupe -CH₂C(O)O-, -C(O)O-, -O-, CH₂O, -NHC(O)NH-, - C(O)NH-, -Ar-(CE₂)_{z}-NHC(O)O-, -Ar-(CE₂)_{z} -NHC(O)NH-, ou -CH₂CH₂-NHC(O)-;
Ar est un groupe aryle divalent;
E est H ou un groupe méthyle;
z est égal à 0 ou 1 ;
k est un entier variant de 0 à 30;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou - C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.
De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.
Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 unités oxydes d'éthylène.
De préférence le ou les monomères associatifs vinyliques représentent de 0,001 à 25 % en poids, de préférence de 0,01 à 15 % en poids et mieux de 0,1 à 10 % en poids du mélange de monomères.

Le ou les monomères tensioactifs vinyliques semi-hydrophobes, éventuellement présents dans le mélange de monomères peuvent modérer les propriétés associatives des polymères associatifs cationiques qui les contiennent, produisant ainsi des gels aqueux ayant une très bonne texture et de très bonnes propriétés rhéologiques.
Par monomère tensioactif vinylique semi-hydrophobe, on entend au sens de la présente invention un monomère qui a une structure similaire à un monomère associatif, mais a une extrémité substantiellement non hydrophobe et ainsi ne confère pas de propriété associative aux polymères.
La propriété d'associativité d'un polymère est liée à la propriété dans un milieu donné, des molécules dudit polymère de s'associer entre elles, ou de s'associer à des molécules d'un co-agent, en général tensioactif, ce qui se traduit dans un certain domaine de concentration par un accroissement de la viscosité du milieu.
Le ou les monomères tensioactifs vinyliques semi-hydrophobes sont généralement des composés ayant deux parties :
(i) un groupe terminal insaturé pour permettre la polymérisation par addition avec les autres monomères du mélange de réaction, et
(ii) un groupe polyoxyalkylène pour atténuer les associations entre les groupes hydrophobes du polymère ou les groupes hydrophobes des autres matériaux éventuellement présents dans la composition comprenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité A peut dériver d'un éther allylique, d'un éther vinylique ou d'un uréthane insaturé non ionique.
L'extrémité A insaturée polymérisable peut aussi dériver d'un acide gras insaturé en C₈-C₃₀ comprenant un ou plusieurs groupe fonctionnel carboxy libre. Ce groupe en C₈-C₃₀ fait partie de l'extrémité insaturée A et est différent des groupes hydrophobes pendant des monomères associatifs, qui sont séparés de l'extrémité insaturée du monomère associatif par un groupe espaceur hydrophile.
La portion B polyoxyalkylène comprend un segment polyoxyalkylène à chaîne longue, qui est essentiellement similaire à la portion hydrophile des monomères associatifs. Des portions polyoxyalkylène B préférées incluent les unités en C₂-C₄ polyoxyéthylène, polyoxypropylène, et polyoxybutylène comprenant de 5 à 250, de préférence de 10 à 100 unités oxyalkylène. Lorsque le monomère tensioactif vinylique semi-hydrophobe comprend plus d'un type d'unité d'oxyalkylène, ces unités peuvent être disposés en séquence aléatoire, non aléatoire, ou à bloc.
De préférence, le ou les monomères tensioactifs vinyliques semi-hydrophobes sont choisis parmi les composés de formules (IV) ou (V) :

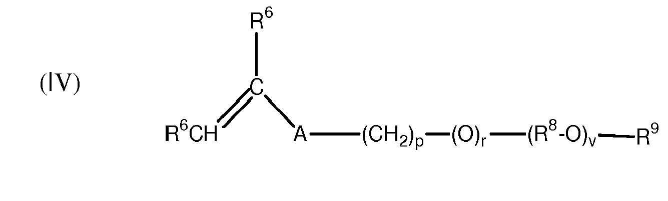

(V) D-A-(CH₂)ₚ-(O)ᵣ-(R⁸-O)ᵥ-R⁹

où, dans chaque formule (IV) ou (V),
chaque R⁶ représente indépendamment H, un alkyle en C₁-C₃₀, -C(O)OH, ou C(O)OR⁷;
R⁷ est un alkyle en C₁-C₃₀;
A est un groupe -CH₂C(O)O-, -C(O)O-, -O-, -CH₂O, -NHC(O)NH-, - C(O)NH-, -Ar-(CE₂)_{z}-NHC(O)O-, -Ar-(CE₂)_{z} -NHC(O)NH-, ou -CH₂CH₂NHC(O)-;
Ar est un groupe aryle divalent ;
E est H ou un groupe méthyle ;
z est égal à 0 ou 1 ;
p est un entier variant de 0 à 30;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄;
D est un alkyle insaturé en C₈-C₃₀ ou un alkyle insaturé en C₈-C₃₀ substitué par un groupe carboxy.
De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H ou

CH₂=CHCH₂O(C₃H₆O)d(C₂H₄O)ₑH;

où
a est égal à 2, 3, ou 4;
b est un entier variant de 1 à 10;
c est un entier variant de 5 à 50;
d est un entier variant de 1 à 10; et
e est un entier variant de 5 à 50.
Des monomères tensioactifs vinyliques semi-hydrophobes préférés sont par exemple les émulsifiants polymérisables commercialisés sous les références EMULSOGEN^{®} R109, R208, R307, RAL109, RAL208 et RAL307 par la société CLARIANT; BX-AA-E5P5 commercialisé par la société BIMAX; et le MAXEMUL^{®} 5010 et 5011 commercialisé par la société UNIQEMA. Les monomères particulièrement préférés sont l'EMULSOGEN^{®} R208, R307 et RAL 307.
Selon les fabricants:
l'EMULSOGEN^{®} R109 est un éther vinylique 1,4-butanediol éthoxylé/propoxylé aléatoire ayant la formule empirique:
CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique:
CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique:
CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :
CH₂=CHCH₂-O(C₃H₆O)₄(C₂H₄O) ₁₀H;
l'EMULSOGEN^{®} RAL 208 qui est un éther allylique éthoxylé/propoxylé aléatoire ayant la formule empirique:
CH₂=CHCH₂-O(C₃H₆O)₄(C₂H₄O)₂₀H;
l'EMULSOGEN^{®} RAL 307 qui est un éther allylique éthoxylé/propoxylé aléatoire ayant la formule empirique :
CH₂=CHCH₂-O(C₃H₆O)₄(C₂H₄O)₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :
CH₂=CHCH₂-O(C₃H₆O)₅(C₂H₄O)₅H.
La quantité du ou des monomères tensioactifs vinyliques semi-hydrophobes utilisés dans la préparation des polymères cationiques (i) utilisés dans la composition selon l'invention peut varier largement et dépend, en autre, des propriétés rhéologiques finales désirées pour le polymère.
Lorsqu'ils sont présents, les monomères tensioactifs vinyliques semi-hydrophobes représentent de 0,01 à 25 % en poids et de préférence de 0,1 à 10% en poids par rapport au poids total du mélange de monomères.

Le ou les polymères cationiques (i) utilisés dans la composition selon l'invention sont préparés à partir d'un mélange de monomères pouvant comprendre un ou plusieurs monomères vinyliques non ioniques hydroxylés.
Ces monomères sont des monomères à insaturation(s) éthylénique(s) comprenant un ou plusieurs substituants hydroxyle.
A titre de monomères vinyliques non ioniques hydroxylés, on peut citer les (méth)acrylates d'alkyle en C₁-C₆ hydroxylés, de préférence les (méth)acrylates d'alkyle en C₁-C₄ hydroxylés, tel que le 2-hydroxyéthylméthacrylate (HEMA), le 2-hydroxyéthyl acrylate (2-HEA), le 3-hydroxypropyl acrylate ; les (méth)acrylamides d'alkyle en C₁-C₄ hydroxylés, tels que le N-(2-hydroxyéthyl) méthacrylamide, le N-(2-hydroxyéthyl) acrylamide, le N-(3-hydroxypropyl) acrylamide, le N-(2,3-dihydroxypropyl) acrylamide ; et leurs mélanges. On peut encore citer l'alcool allylique, le glycérol monoallyl éther, le 3-méthyl-3-butèn-1-ol, les précurseurs de l'alcool vinylique et leurs équivalents, tel que l'acétate de vinyle.
Lorsqu'il(s) est (sont) présent(s), le ou les monomères vinyliques non ioniques hydroxylés représentent généralement jusqu'à 10% en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques utilisés (i) dans la composition selon l'invention sont préparés à partir d'un mélange de monomères qui peut comprendre un ou plusieurs monomères réticulants permettant d'introduire des ramifications et de contrôler la masse moléculaire.
Des agents réticulants poly-insaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulant utilisables peuvent être des monomères polyfonctionnels comprenant des groupes réactifs multiples tels que des groupes époxydes, isocyanates et des groupes silanes hydrolysables. De nombreux composés poly-insaturés peuvent être utilisés pour générer un réseau tri-dimensionnel partiellement ou substantiellement réticulé.
Des exemples de monomères réticulants polyinsaturés utilisables sont par exemple les monomères aromatiques polyinsaturés, tel que le divinylbenzène, le divinyl naphtylène, et le trivinylbenzène ; les monomères alicycliques polyinsaturés, tel que le 1,2,4-trivinylcyclohexane; les esters difonctionnels de l'acide phthalique, tel que le diallyl phthalate ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène, et le 1,5 heptadiène.
D'autres monomères réticulants polyinsaturés utilisables sont par exemple les éthers polyalcényles, tels que le triallyl pentaérythritol, le diallyl pentaérythritol, le diallyl saccharose, l'octaallyl saccharose et le triméthylolpropane diallyl éther ; les esters polyinsaturés de polyalcools ou de polyacides, tel que le 1,6-hexanediol di(méth)acrylate, le tétraméthylène tri(méth)acrylate, l'allyl acrylate, le diallyl itaconate, le diallyl fumarate, le diallyl maléate, le triméthylolpropane tri(méth)acrylate, le triméthylolpropane di(méth)acrylate et le polyéthylène glycol di(méth)acrylate, les alkylène bisacrylamides, tel que le méthylène bisacrylamide, le propylène bisacrylamide; les dérivés hydroxylés et carboxylés du méthylène bisacrylamide, tel que le N,N'-bisméthylol méthylène bisacrylamide ; les polyéthylèneglycol di(méth)acrylates, tels que l'éthylèneglycol di(méth)acrylate, le diiéthylèneglycol di(méth)acrylate, le triéthylèneglycol di(méth)acrylate, les silanes polyinsaturés tels que le diméthyldivinylsilane, le méthyltrivinylsilane, l'allyldiméthylvinylsilane, le diallyldiméthylsilane et le tétravinylsilane, les stannanes polyinsaturés, tel que le tétraallyl étain et le diallyldiméthyl étain.
Des monomères réticulants monoinsaturés utilisables et portant un groupe réactif peuvent être les N-méthylolacrylamides; les N-alkoxy(méth)acrylamides, où le groupe alkoxy est un groupe C₁-C₁₈; et les silanes hydrolysables insaturés tel que triéthoxyvinylsilane, le tris-isopropoxyvinylsilane, et le 3-triéthoxysilylpropyl méthacrylate.
Des monomères réticulants polyfonctionnels utilisables et comprenant plusieurs groupes réactifs peuvent être par exemple les silanes hydrolysables tel que l'éthyltriéthoxysilane et l'éthyltriméthoxysilane; les silanes hydrolysables époxylés, tel que le 2-(3,4-époxycyclohexyl)éthyltriéthoxysilane et le 3-glycidoxypropyltriméthyoxysilane; les polyisocyanates, tels que le 1,4-diisocyanatobutane, le 1,6-diisocyanatohexane, le 1,4-phénylènediisocyanate, et le 4,4'-oxybis(phénylisocyanate); les époxides insaturés, tel que le glycidyl méthacrylate et l'allylglycidyl éther; les polyépoxides, tel que le diglycidyl éther, le 1,2,5,6-diépoxyhexane, et l'éthylèneglycoldiglycidyl éther.
Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel que un vinyl éther, un allyl éther, un ester acrylate ou un ester méthacrylate. De tels monomères réticulants peuvent être par exemple le diméthacrylate éthoxylé de biphénol A, le diméthacrylate éthoxylé de biphénol F, et le triméthylol propane triméthacrylate éthoxylé.
D'autres monomères réticulants éthoxylés utilisables dans la présente invention sont par exemple les agents réticulants dérivés des polyols éthoxylés divulgués dans le brevet US 6 140 435.
Des exemples particulièrement préférés de monomères réticulants sont des esters acrylates et méthacrylates de polyols ayant au moins deux groupes ester acrylate ou méthacrylate, tel que le triméthylolpropane triacrylate (TMPTA), le triméthylolpropane diméthacrylate, le triéthylène glycol diméthacrylate (TEGDMA), et le diméthacrylate de bisphénol A éthoxylé (30) (EOBDMA).
Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur variant de 0,001 à 5% en poids, de préférence de 0,05 à 2% en poids, mieux de 0,1 à 1% en poids par rapport au poids total du mélange de monomères.

Le mélange de monomères peut comprendre un ou plusieurs agents de transfert de chaîne. Les agents de transferts de chaîne sont des composés bien connus de l'état de la technique.
On peut citer en particulier les composés thiolés, les composés disulfures, tels que les C₁-C₁₈ mercaptans, les acides mercaptocarboxyliques, les esters d'acides mercaptocarboxyliques, les thioesters, les C₁-C₁₈ alkyl disulfures, les aryldisulfures, les thiols polyfonctionnels ; les phosphites et hypophosphites ; les composés haloalkyl, tel que le tétrachlorure de carbone, le bromotrichlorométhane; et les agents de transfert de chaîne insaturés, tel que l'alphaméthylstyrène.
Les thiols polyfonctionnels sont par exemple les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate); les thiols hexafonctionnels, tel que le pentaérytritol-hexa-(thioglyconate).
De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.
De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.
Lorsqu'il(s) est (sont) présents, le ou les agents de transferts de chaîne sont ajoutés au mélange de monomères de préférence jusqu'à 10% en poids par rapport au poids total du mélange de monomère. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un ou plusieurs stabilisateurs polymériques pour l'obtention de dispersions ou d'émulsions stables. De préférence, les polymères sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine; les polymères naturels modifiés, tels que la méthylcellulose, l'hydroxypropylcellulose, la carboxyméthylcellulose, les hydroxyéthylcelluloses allyliques.
Les stabilisateurs polymériques sont utilisés en une quantité au plus égale à 2 % en poids par rapport au poids total de l'émulsion, de préférence en une quantité comprise 0,0001 et 1 % en poids, mieux entre 0,01 et 0,5 % en poids par rapport au poids du mélange de monomères.

Selon un mode de réalisation préféré, le mélange de monomères comprend, par rapport au poids total du mélange de monomères :
a) de 10 à 70 % en poids du ou des monomères vinyliques substitués par un ou plusieurs groupes amino,
b) de 20 à 80 % en poids du ou des monomères vinyliques non ioniques hydrophobes,
c) de 0,001 à 25 % en poids du ou des monomères vinyliques associatifs,
d) de 0 à 25 % en poids du ou des monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs stabilisateurs polymériques.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10 % en poids du ou des agents de transfert de chaîne,
h) de 0 à 2 % en poids du ou des stabilisateurs polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisis parmi:
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6 (1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes:

   CH₂=CH-O(CH₂)ₐO(C₃H₆0)_{b}(C₂H₄O)_{c}H

   ou

   CH₂=CHCH₂O(C₃H₆O)_{d}(C₂H₄O)ₑH;

   où
   a est égal à 2, 3, ou 4;
   b est un entier variant de 1 à 10;
   c est un entier variant de 5 à 50;
   d est un entier variant de 1 à 10; et
   e est un entier variant de 5 à 50.
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs stabilisateurs polymériques.

Les polymères cationiques (i) préférés selon l'invention sont des polymères issus de la polymérisation du mélange de monomères suivants:
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination CARBOPOL AQUA CC POLYMER et qui correspond à la dénomination INCI POLYACRYLATE-1 CROSSPOLYMER.
Le POLYACRYLATE-1 CROSSPOLYMER est le produit de la polymérisation d'un mélange de monomères comprenant (ou constitué de):
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1% en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.
Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyles sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (initiateur) tel que le persulfate de sodium, ou tout autre catalyseur de polymérisation par addition adaptée, comme cela est bien connu dans le domaine des polymères. La réaction est agitée jusqu'à ce que la polymérisation soit complète, généralement pendant une durée variant de 4 heures à 16 heures. L'émulsion de monomères peut être chauffée à une température comprise entre 20 et 80°C avant l'addition de l'initiateur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en solides qui varie entre 10 et 40 % en poids. Généralement, la quantité totale de polymères dans l'émulsion obtenue varie entre 15 et 35% en poids, en général au plus 25 % en poids.

Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.
Tous types de tensioactifs non ioniques, anioniques, amphotères ou cationiques classiquement utilisés dans les polymérisations en l'émulsion peuvent être utilisés.
La polymérisation peut être effectuée en présence d'un ou plusieurs initiateurs de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).

Les cyclodextrines sont notamment des oligosaccharides de formule (VI): 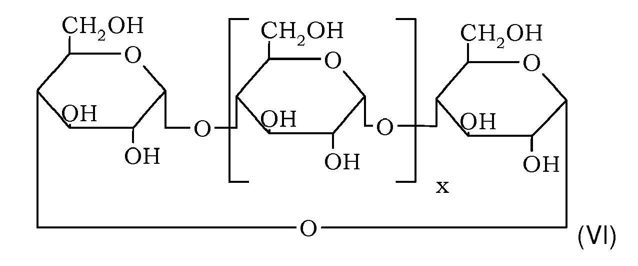 dans laquelle x peut être un nombre égal à 4 (ce qui correspond à l'α-cyclodextrine), à 5 (β-cyclodextrine) ou à 6 (γ-cyclodextrine).

On peut notamment utiliser une beta-cyclodextrine vendu par la société WACKER sous la dénomination CAVAMAX W7 et une gamma cyclodextrine vendu par la société WACKER sous la dénomination CAVAMAX W8.
Par cyclodextrines au sens de la présente invention, on entend aussi les dérivés substitués de cyclodextrines comme par exemple les méthyl cyclodextrines en particulier la méthyl-beta-cyclodextrine commercialisée par la société WACKER sous la dénomination CAVASOL W7.

De préférence, les cyclodextrines de l'invention sont non substitués. Encore plus préférentiellement, on utilisera une beta-cyclodextrine.

Selon l'invention, la ou les cyclodextrines peuvent représenter de 0,2% à 30 % en poids, de préférence de 0,5 % à 15 % en poids, préférentiellement de 1 % à 10 % en poids, et mieux de 1,5 à 5% en poids du poids total de la composition finale.

Les compositions de l'invention comprennent en outre un ou plusieurs tensioactifs qui est généralement présent en une quantité allant de 0,2% à 40% en poids environ, de préférence de 1% à 35% et encore plus préférentiellement de 1,5 % à 30%, par rapport au poids total de la composition.

La cyclodextrine et le tensioactif sont présents de préférence à une concentration efficace pour nacrer la composition et/ou pour former un complexe insoluble dans la composition entre la cyclodextrine et le tensioactif ou les tensioactifs.

Le rapport tensioactif/cyclodextrine peut varier de 0,01 à 300, de préférence de 0,1 à 100 et plus particulièrement de 0,3 à 25.

Les tensioactifs convenant à la mise en oeuvre de la présente invention peuvent être de toute nature et de préférence sont solubles dans l'eau à température ambiante :

### (i) Tensioactif(s) anionique(s):

A titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides et les esters d'acides gras du sorbitan oxyéthylénés, en particulier de 2 à 10 moles d'oxyde d'éthylène constituent des tensioactifs non-ioniques rentrant particulièrement bien dans le cadre de la présente invention.
Dans une variante préférée de l'invention, les compositions comprennnent du laurate de sorbitan oxyéthyléné à 4 moles d'oxyde d'éthylène (TWEEN 21 de UNIQEMA).

### (iii) Tensioactif(s) amphotère(s) ou zwittérionique(s):

Les agents tensioactifs amphotères ou zwittérioniques peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂ -CONHCH₂CH₂ -N(R₃)(R₄)(CH₂COO-) (1)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle;
et

R_{2'}-CONHCH₂CH₂-N(B)(C) (2)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphodiacetate commercialisé sous la dénomination commerciale MIRANOL C2M concentré NP par la Société RHODIA CHIMIE.

### (iv) Tensioactifs cationiques:

Les tensioactifs cationiques peuvent être choisis parmi :
A) les sels d'ammonium quaternaires de la formule générale (VII) suivante 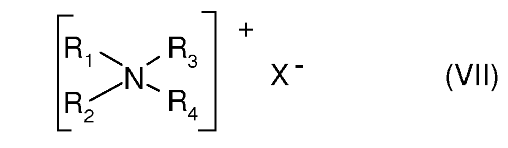 dans laquelle X⁻ est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
         De préférence le tensioactif cationique est un sel (par exemple chlorure) de cétyl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate;
   De préférence le tensioactif cationique est un sel (par exemple chlorure) de stéaramidopropyl diméthyl (myristylacétate) ammonium.
B)- les sels d'ammonium quaternaire de l'imidazolinium, comme par exemple celui de formule (VIII) suivante : 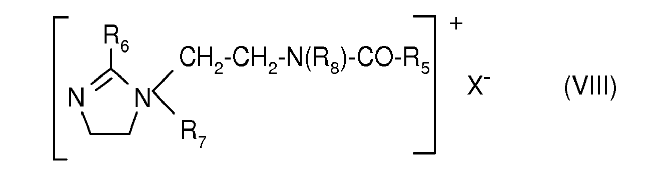 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C)- les sels de diammonium quaternaire de formule (IX): 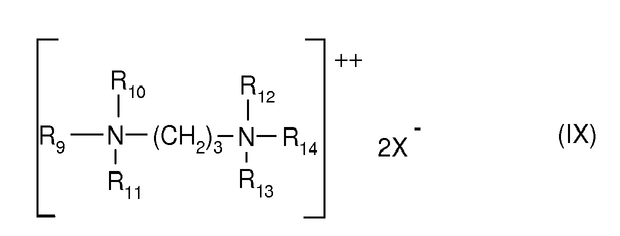 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D)- les sels d'ammonium quaternaire contenant au moins une fonction ester de formule (X) suivante : 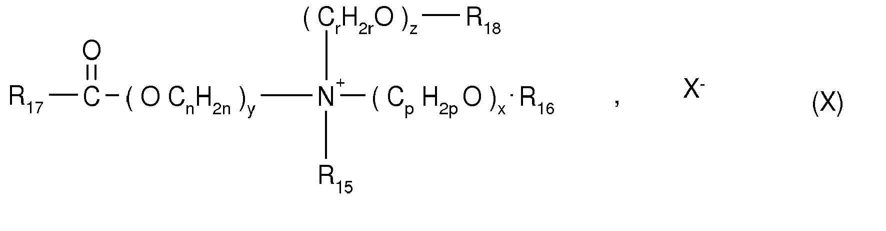 dans laquelle :
   - R₁₅ est choisi parmi les radicaux alkyles en C₁-C₆ et les radicaux hydroxyalkyles ou dihydroxyalkyles en C₁-C₆;
   - R₁₆ est choisi parmi :
   - le radical 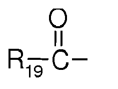
   - les radicaux R₂₀ hydrocarbonés en C₁-C₂₂ linéaires ou ramifiés, saturés ou insaturés,
   - l'atome d'hydrogène,
   - R₁₈ est choisi parmi :
   - le radical 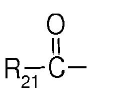
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés;
   - n, p et r, identiques ou différents, sont des entiers valant de 2 à 6;
   - y est un entier valant de 1 à 10 ;
   - x et z, identiques ou différents, sont des entiers valant de 0 à 10;
   - X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (X) dans laquelle :
- R₁₅ désigne un radical méthyle ou éthyle,
- x et y sont égaux à 1;
- z est égal à 0 ou 1 ;
- n, p et r sont égaux à 2 ;
- R₁₆ est choisi parmi :
- le radical 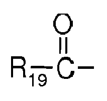
- les radicaux méthyle, éthyle ou hydrocarbonés en C₁₄-C₂₂
- l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
- le radical 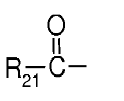
- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA-WITCO.

Parmi les sels d'ammonium quaternaire on préfère le chlorure de cétyltriméthylammonium, ou encore, le chlorure palmitamidopropyl triméthyl ammonium commercialisé sous la dénomination VARISOFT PA TC par la société DEGUSSA.

Les compositions comprennent de préférence au moins un tensioactif anionique. Ce ou ces tensioactifs anioniques sont généralement présents dans des concentrations allant de 3 à 30% en poids par rapport au poids total de la composition, plus particulièrement de 5 à 20 % en poids.

On utilise de préférence comme agent tensioactif anionique les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodiacetate ou sodiumcocoamphopropionate commercialisés notamment par la société RHODIA CHIMIE sous la dénomination commerciale "MIRANOL^{®} C2M CONCNP" en solution aqueuse à 38 % de matière active ou sous la dénomination MIRANOL^{®} C32;
- soit un agent tensioactif amphotère tel que les alkylbétaïnes en particulier la cocobétaïne commercialisée sous la dénomination "DEHYTON^{®} AB 30" en solution aqueuse à 32 % de MA par la société COGNIS ou tel que les alkyl (C₈-C₂₀) amidoalkyl (C₁-C₆) bétaïnes en particulier la TEGOBETAINE^{®} F 50 commercialisée par la société DEGUSSA.

Les tensioactifs amphotères ou non ioniques sont de préférence présents dans des concentrations allant de 0,5 à 20% en poids et de préférence de 1 à 15% en poids par rapport au poids total de la composition.

Selon une variante préférée de l'invention, les compositions cosmétiques peuvent également comprendre un ou plusieurs agents de conditionnement des matières kératiniques.

Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques, les polymères cationiques autres que ceux de l'invention, les silicones, les huiles minérales, végétales ou animales, les céramides, les pseudocéramides et leurs mélanges.

Les polyoléfines sont de préférence des poly-α-oléfines et en particulier:
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence allant de 1000 à 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par:
- les hydrocarbures, tels que l'hexadécane et l'huile de paraffine;

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société NALCO, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).
On peut également utiliser les polymères qui sont constitués de motifs récurrents répondant à la formule : 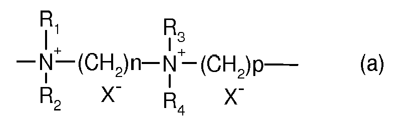 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 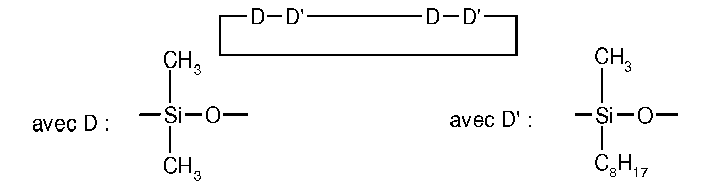 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société DEGUSSA qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles MIRASIL DPDM de RHODIA CHIMIE ;
- les huiles des séries RHODORSIL 70 633 et 763 de RHODIA CHIMIE;
- l'huile DOW CORNING 556 COSMETIC GRAD FLUID de DOW CORNING ;
- les silicones de la série PK de BAYER comme le produit PK20;
- les silicones des séries PN, PH de BAYER comme les produits PN1000 et PH1000;
- certaines huiles des séries SF de GENERAL ELECTRIC telles que SF 1023,
SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants:
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'une gomme de polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société DEGUSSA ;
- des groupements hydroxylés comme les polyorganosiloxanes à fonction hydroxyalkyle décrits dans la demande de brevet français FR-A-85 16334.
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société DEGUSSA sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle;
b) 0 à 40% en poids d'acide acrylique ;
c) 5 à 40% en poids de macromère siliconé de formule: 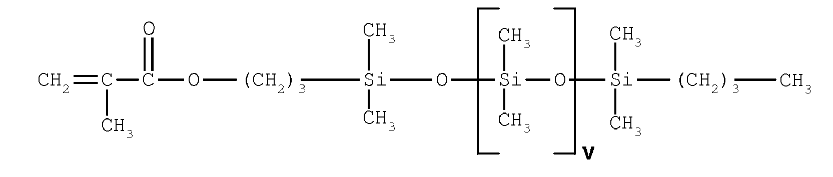 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE
- les polysiloxanes à groupements aminés tels que les amodiméthicones ou les triméthylsilylamodiméthicones;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique . - le N-docosanoyl N-méthyl-D-glucamine
ou les mélanges de ces composés.

Les esters gras liquides (ii) utilisables dans la présente invention sont de préférence monomériques. Ils sont de préférence non ioniques et non siliconés.

Selon l'invention, par esters gras liquides on entend un ester qui est liquide à température ambiante (25°C) et à la pression atmosphérique (1atm) et qui est issu d'un acide carboxylique et d'un alcool, l'un au moins de ces deux constituants comportant au moins 7 atomes de carbone.

Les esters gras liquides selon l'invention sont notamment choisis parmi:
1) les esters d'acide carboxylique en C3-C30 et d'alcool en C1-C30, l'un au moins de l'acide ou de l'alcool étant ramifié ou insaturé (possédant au moins une double-liaison carbone-carbone), et
2) les esters d'acide aromatique en C7-C30 dont la fonction carboxylique est directement liée au cycle aromatique et d'alcool en C1-C30,

De préférence les esters selon l'invention sont des esters liquides insolubles dans l'eau.
Les esters d'acides carboxyliques liquides insolubles dans l'eau sont insolubles dans l'eau à une concentration supérieure ou égale à 0,1% en poids dans l'eau à 25°C, c'est à dire qu'ils ne forment pas, dans ces conditions, une solution macroscopiquement isotrope transparente.

Le nombre total de carbone des esters de l'invention est généralement supérieur ou égal à 10 et de préférence inférieure à 50 et plus particulièrement inférieur à 25.

Les esters liquides d'acide carboxylique en C3-C30 et d'alcool en C1-C30, l'un au moins de l'acide ou de l'alcool étant ramifié ou insaturé, sont notamment choisis parmi les esters d'acide carboxylique en C6-C24 et d'alcool en C3-C20.
Les esters selon l'invention sont notamment choisis parmi :
les esters d'acide carboxylique linéaire ayant de 12 à 26 atomes de carbone et d'alcool ramifié ayant de 3 à 12 atomes de carbone,
les esters d'acide carboxylique linéaire ayant de 2 à 12 atomes de carbone et d'alcool ramifié ayant de 8 à 26 atomes de carbone.
les esters d'acide carboxylique ramifié ayant de 8 à 26 atomes de carbone, de préférence 8 à 12 et d'alcool ramifié ayant de 8 à 26 atomes de carbone, de préférence 8 à 12.

On peut citer le béhénate d'octyldodécyle ; le béhénate d'isocétyle ; le lactate d'isocétyle ; le lactate d'isostéaryle ; le lactate de linoléyle ; le lactate d'oléyle ; l'octanoate de isostéaryle ; l'octanoate d'isocétyle ; l'oléate de décyle; l'isostéarate d'isocétyle; le laurate d'isocétyle ; le stéarate d'isocétyle; l'octanoate d'isodécyle; l'oléate d'isodécyle ; l'isononanoate d'isononyle ; le palmitate d'isostéaryle; l'isostéarate de myristyle; l'isononanoate d'octyle; l'isononate de 2-éthylhexyle; l'isostéarate d'octyle; l'érucate d'octyldodécyle ; les palmitates d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, l'isostéarate de butyle, le stéarate d'isobutyle ; le laurate de 2-hexyldécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.
On peut notamment citer : le sébacate de diisopropyle ; l'adipate de diisopropyle ; l'adipate de diisostéaryle; le stéarate d'octyldodécyl stéaroyl; le tétraisononanoate de pentaérythrityle; le tétraisostéarate de pentaérythrityle; le citrate de triisopropyle ; le citrate de triisotéaryle; le citrate de trioctyldodécyle.

On peut également utiliser les esters liquides d'acide carboxylique ramifié ayant de 4 à 6 atomes de carbone et d'alcool ayant de 8 à 26 atomes de carbone.

Ces esters liquides ramifiés selon l'invention ont de préférence la formule suivante :

R₁ COOR₂ (VI)

dans laquelle :
R₁ désigne un radical hydrocarboné, ramifié, éventuellement mono ou polyhydroxylé, ayant de 3 à 5 atomes de carbone,
R₂ désigne un radical hydrocarboné, linéaire ou ramifié, éventuellement mono ou polyhydroxylé, ayant de 12 à 26 atomes de carbone, de préférence ayant de 16 à 22 atomes de carbone.

R₁ désigne de préférence un radical alkyle ramifié ayant de 3 à 5 atomes de carbone, et plus particulièrement un radical tertio-butyle.
R₂ désigne de préférence un radical alkyle saturé ou insaturé ayant 12 à 26 atomes de carbone, plus particulièrement ramifié et encore plus particulièrement choisi parmi les radicaux tridécyle, isocétyle, isostéaryle, octyldodécyle et isoarachidyle.

Les esters liquides ramifiés particulièrement préférés sont le néopentanoate d'isostéaryle (formule (VI) dans laquelle R₁=tertio-butyle et R₂=isostéaryle), le néopentanoate de tridécyle, le néopentanoate d'isocétyle, et le néopentanoate d'isoarachidyle.

De préférence, l'acide et l'alcool de l'ester sont saturés. De préférence l'alcool est un monoalcool (une seule fonction hydroxyle).

Parmi les esters cités ci-dessus, on préfère utiliser le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, le isostéarate de butyle, le stéarate d'isobutyle ; le laurate de 2-hexyldécyle, le néopentanoate d'isostéaryle, néopentanoate de tridécyle et l'isononanate d'isononyle.

Les esters d'acide aromatique en C7-C30 et d'alcool en C1-C30 sont de préférence des esters d'acide aromatique en C7-C17 et d'alcool en C1-C20. Ces esters sont notamment les benzoates d'alkyle en C12-C15, le benzoate d'isostéaryle, le benzoate d'octyledodécyle, le benzoate de béhényle, le benzoate d'éthyl-2hexyle.

Parmi les esters cités ci-dessus, on préfère encore plus particulièrement utiliser le palmitate d'isopropyle, le palmitate d'éthyl-2-héxyle, les myristates d'isopropyle, de t-butyle, l'isostéarate de butyle, le stéarate d'isobutyle ;l'isononanate d'isononyle et le néopentanoate d'isostéaryle.

Les esters selon l'invention sont de préférence des monoesters d'acide carboxylique et de monoalcool.

Selon l'invention, les agents conditionneurs peuvent représenter de 0,001 % à 10 % en poids, de préférence de 0,005 % à 5 % en poids, et encore plus préférentiellement de 0,01 % à 3 % en poids, du poids total de la composition finale.

Le milieu physiologiquement acceptable est de préférence constitué par de l'eau ou un mélange d'eau et de solvants cosmétiquement ou dermatologiquement acceptables tels que des monoalcools, des polyols, des éthers de polyols qui peuvent être utilisés seuls ou en mélange. L'eau représente de préférence de 30 à 98% en poids et de préférence de 50 à 98% en poids par rapport au poids total de la composition.

On peut citer plus particulièrement les monoalcools tels que l'éthanol, l'isopropanol, les polyols tels que le diéthylèneglycol, la glycérine et les éthers de polyols tels que les éthers de diéthylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les acides gras, les épaississants, les parfums, les conservateurs, les filtres solaires, les protéines, les vitamines et provitamines, les polymères anioniques, non ioniques ou amphotères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants, des produits démaquillants.

La quantité minimale en tensioactif est alors celle juste suffisante pour conférer à la composition finale un pouvoir moussant et/ou détergent satisfaisant.

Ainsi, selon l'invention, le tensioactif détergent peut représenter de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes:
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Lorsque la composition se présente sous la forme d'un après-shampooing éventuellement à rincer, elle contient avantageusement un tensioactif cationique, sa concentration allant généralement de 0,1 à 10% en poids et de préférence de 0,5 à 5% en poids par rapport au poids total de la composition.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLES 1 A 3

On a préparé des shampooings de composition suivante :

| | 1 | 2 | 3 |
|---|---|---|---|
| Lauryl éther sulfate de sodium (2.2 OE) en solution aqueuse (TEXAPON AOS 225 UP de COGNIS) | 15,5 gMA | 15,5 gMA | 14 gMA |
| Cocoyl amidopropyl bétaine en solution aqueuse (TEGO BETAIN F 50 de GOLDSCHMIDT) | 2,4 gMA | 2,4 gMA | 2,4 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC POLYMER de NOVEON) | 0,6 gMA | 0,6 gMA | 0,6 gMA |
| Chlorure d'hydroxypropyl guar triméethyl ammonium (JAGUAR C13S de RHODIA) | 0,1 g | 0,2 g | |
| Bêta - cyclodextrine (cyclomaltoheptaose) (CAVAMAX W7 PHARMA) | 1,72 gMA | 1,72 gMA | 1,72 gMA |
| Poly diméthylsiloxane (DC 200 FLUID 300.000 de DOW CORNING) | 2,7 g | | |
| Poly diméthylsiloxane (DC 200 FLUID 60.0000 de DOW CORNING) | | 2,7 | |
| Poly diméthylsiloxane (DC 200 FLUID 500.0000 de DOW CORNING) | | | 1,5 g |
| Hydroxyéthylcellulose quaternisée (POLYMER JR 400 de AMERCHOL) | | | 0,4 g |
| Alcool cétylique oxyéthyléné (20 OE) oxypropyléné (5 OP) (PROCETYL AWS de CRODA) | 0,5 | | |
| Monolaurate de sorbitan oxyéthyléné à 4 mole d'oxyde d'éthylène | | 6g | 6g |
| Cire de jojoba oxyéthylenée (120 OE) (FLORASOLVS JOJOBA PEG 120 de FLORATECH) | 0,05 g | | |
| Néopentanoate d'isostéaryle (CERAPHYL 375 de ISP) | 0,25 g | | |
| Chlorure de sodium | 2,2 g | 1g | 1g |
| Acide lactique | 0,29 | 0,34 g | 0,28 g |
| Sel di-sodique de fuchsine acide D (CI: 17200) | 0,00012 g | | |
| Acide salicylique en poudre (conservateur) | 0,2 g | 0,2 g | 0,2 g |
| P-hydroxybenzoate d'ethyle (conservateur) | 0,15g | 0,15g | 0,15g |
| Benzoate de sodium (conservateur) | 0,5 g | 0,5 g | 0,5 g |
| P-hydroxybenzoate de methyle, sel de sodium (conservateur) | 0,4 g | 0,4 g | 0,4 g |
| Hydrolysat de conchyoline (pm: 600) dans l'eau à3% | 0,02 g | | |
| Parfum | 0,5 g | 0,5 g | |
| Hydroxyde de sodium pur ou Acide citrique, 1 H2O | qs pH 5,3 | qs pH 5,3 | qs pH 5,3 |
| Eau desionisée qsp | 100 g | 100 g | 100 g |

Ces compositions ont un bel effet nacré, sont stables et présentent de bonnes propriétés cosmétiques.

### EXEMPLES 4 ET 5

On prépare des shampooings de composition suivante :

| | 4 | 5 |
|---|---|---|
| Lauryl éther sulfate de sodium (2.2 OE) en solution aqueuse (TEXAPON AOS 225 UP de COGNIS) | | 15,5 gMA |
| Lauryl sulfate de sodium en solution aqueuse (TEXAPON A 795 de COGNIS) | 15,5 gMA | |
| Cocoyl amidopropyl bétaine en solution aqueuse (TEGO BETAIN F 50 de GOLDSCHMIDT) | 2,4 gMA | |
| Cocoyl bétaine en solution aqueuse (DEHYTON AB 30 de COGNIS) | | 2,4 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC POLYMER de NOVEON) | 0,6 gMA | 0,6 gMA |
| Chlorure d'hydroxypropyl guar triméethyl ammonium (JAGUAR C13S de RHODIA) | 0,1 g | 0,1 g |
| Bêta - cyclodextrine (cyclomaltoheptaose) (CAVAMAX W7 PHARMA) | 1,72 gMA | 1,72 gMA |
| Poly diméthylsiloxane (DC 200 FLUID 300.000 de DOW CORNING) | 2,7 g | 2,7 g |
| Chlorure de sodium | 1,2 g | 1,2 g |
| Acide lactique | 0,28 | 0,28 g |
| Conservateurs | qs | qs |
| Parfum | 0,5 g | 0,5 g |
| Hydroxyde de sodium pur ou Acide citrique, 1 H2O | qs pH 5,3 | qs pH 5,3 |
| Eau desionisée qsp | 100 g | 100 g |

Ces compositions ont un bel effet nacré, sont stables et présentent de bonnes propriétés cosmétiques.

### EXEMPLE 6

On prépare un shampooing de composition suivante :

| | 6 |
|---|---|
| Lauryl éther sulfate de sodium (2.2 OE) en solution aqueuse (TEXAPON AOS 225 UP de COGNIS) | 14 gMA |
| Cocoyl bétaine en solution aqueuse (DEHYTON AB 30 de COGNIS) | 2 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC POLYMER de NOVEON) | 0,6 gMA |
| Myristate d'isopropyle | 1,5 g |
| Bêta - cyclodextrine (cyclomaltoheptaose) (CAVAMAX W7 PHARMA) | 1,72 gMA |
| Hydroxyéthylcellulose quaternisée (POLYMER JR 400 de AMERCHOL) | 0,5 g |
| 2-oléamido-1,3-octanediol | 0,01 g |
| Chlorure de sodium | 1,2 g |
| Acide lactique | 0,28 g |
| Conservateurs | qs |
| Parfum | qs |
| Hydroxyde de sodium pur ou Acide citrique, 1 H2O | qs pH 5,3 |
| Eau desionisée | Qsp 100 g |

Cette composition a un bel effet nacré, est stable et présente de bonnes propriétés cosmétiques.

### EXEMPLE 7

On a préparé les 2 compositions suivantes :

| % en matière active | A | B (invention) |
|---|---|---|
| Polyquaternium-10 (JR400 de RHODIA) | 0,4 | 0,4 |
| Copolymère de C10-C30 alkyl PEG itaconate, C1-C4 alkylaminoacrylate, d'acide acrylique et méthacrylique (Structure Plus de National Starch) | 0,2 | - |
| Copolymère d'esters d'acide acrylique ou méthacrylique, de méthacrylate de C1-4 | - | 0,2 |
| dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC POLYMER de LUBRIZOL | | |
| Néopentanoate d'isostéaryle (Ceraphyl 375 - ISP) | 0,5 | 0,5 |
| Béta-cyclodextrine (Cavamax W7 - Wacker) | 2 | 2 |
| Lauryléther sulfate de Na 2,2 moles d'OE | 14 | 14 |
| Cocamidopropyl bétaine | 2,4 | 2,4 |
| Dimethicone (Mirasil DM 500000 - Rhodia) | 1,5 | 1,5 |
| Conservateur | 0,45 | 0,45 |
| Agent de pH | Qs pH 6,8-7 | Qs pH 6,8-7 |
| Eau | Qs 100 % | Qs 100 % |

On a appliqué 6 g de la composition A sur une demi-tête d'un modèle, on appliqué 6 g de composition B sur l'autre demi-tête. Les compositions ont été rincées puis un expert a évalué la souplesse des cheveux. Ces opérations ont été répétées sur 10 modèles.
Dans 90% des cas, la souplesse des cheveux traités avec la composition B est supérieure à celle des cheveux traités avec la composition A.

## Revendications

1. Composition cosmétique **caractérisée en ce qu'**elle comprend dans un milieu aqueux :
(i)- un ou plusieurs polymères cationiques qui sont produits par polymérisation d'un mélange de monomères comprenant :
a) un ou plusieurs monomères vinyliques substitués par un ou plusieurs groupes amino,
b) un ou plusieurs monomères vinyliques non ioniques hydrophobes, choisis parmi ceux de formules (I) et (II) :
(I) CH₂=C(X)Z,
(II) CH₂=CH-OC(O)R;
dans lesquelles formules (I) et (II) :
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ; chaque R¹ représente indépendamment un groupe alkyle en C₁-C₃₀ , un groupe alkyle en C₂-C₃₀ hydroxylé, ou un groupe alkyle en C₁-C₃₀ halogéné et
c) un ou plusieurs monomères vinyliques associatifs,
e) un ou plusieurs monomères vinyliques non ioniques hydroxylés,
(ii)- une ou plusieurs cyclodextrines ou l'un de ses dérivés, et
(iii)-un ou plusieurs tensioactifs.

2. Composition selon la revendication 1, **caractérisé en ce que** le ou les monomères vinyliques substitués par un ou plusieurs groupes amino sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C1-C4)alkylamino(C₁-C₈)alkyl(méth)acrylamides,
- les (méth)acrylamides hétérocycliques comprenant un atome d'azote,
- les (méth)acrylates hétérocycliques comprenant un atome d'azote,
et leurs mélanges.

3. Composition selon la revendication 2 **caractérisé en ce que** le ou les monomères vinyliques substitués par un ou plusieurs groupes amino sont choisis parmi :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

4. Composition selon l'une quelconque des revendications 1 à 3, **caractérisée en ce que** le ou les monomères vinyliques substitués par un ou plusieurs groupes amino représentent généralement de 10 à 70 % en poids, de préférence de 20 à 60 % en poids, par rapport au poids total du mélange de monomères.

5. Composition selon l'une quelconque des revendications précédentes, **caractérisée en ce que** le ou les monomères vinyliques non ioniques hydrophobes sont choisis parmi les (méth)acrylates d'alkyle en C₁-C₃₀, les (alkyl en C₁-C₃₀)(méth)acrylamides, le styrène, les styrènes substitués, les esters de vinyle, les nitriles insaturés, et les silanes insaturés.

6. Composition selon l'une quelconque des revendications précédentes, **caractérisée en ce que** le ou les monomères vinyliques non ioniques hydrophobes représentent généralement de 20 à 80% en poids, de préférence de 20 à 70% en poids, par rapport au poids total du mélange de monomères.

7. Composition selon l'une quelconque des revendications précédentes, **caractérisée en ce que** le ou les monomères vinyliques associatifs sont choisis parmi les composés de formule (III) : 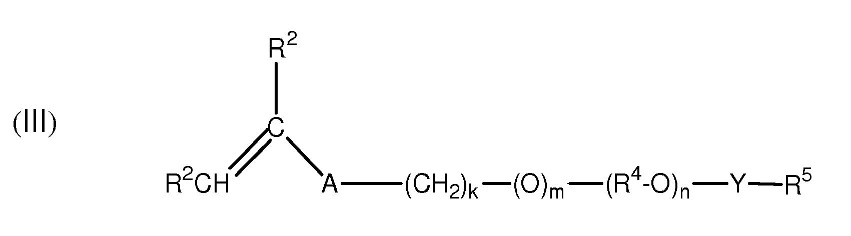 dans laquelle
chaque R² est indépendamment H, un groupe méthyle, un groupe
-C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, - C(O)NH-, -Ar-(CE₂)₂-NHC(O)O-, -Ar-(CE₂)_{z} -NHC(O)NH-, ou -CH₂CH₂-NHC(O)-;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R4^{N}HC(O)NH-, ou - C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.

8. Composition selon la revendication 7, **caractérisée en ce que** le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.

9. Composition selon l'une quelconque des revendications précédentes, **caractérisée en ce que** le ou les monomères vinyliques associatifs représentent de 0,001 à 25 % en poids, de préférence de 0,01 à 15 % en poids du mélange de monomères.

10. Composition selon l'une quelconque des revendications précédentes, **caractérisée en ce que** le mélange de monomères comprend un ou plusieurs monomères tensioactifs vinyliques semi-hydrophobes d) choisis parmi les composés de formules (IV) ou (V) : 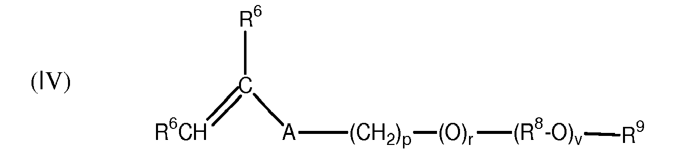
(V) D-A-(CH₂)ₚ-(O)ᵣ-(R⁸-O)ᵥ-R⁹
où, dans chaque formule (IV) ou (V),
chaque R⁶ représente indépendamment H, un alkyle en C₁-C₃₀, - C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, - C(O)NH-, -Ar-(CE₂)_{z}-NHC(O)O-, -Ar-(CE₂)_{z} -NHC(O)NH-, ou -CH₂CH₂NHC(O)- ;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alcényle en C₈-C₃₀ ou un alcényle en C₈-C₃₀ substitué par un groupe carboxy.

11. Composition selon la revendication 10 **caractérisée en ce que** le mélange de monomères comprend un ou plusieurs monomères tensioactifs vinyliques semi-hydrophobes ayant l'une des formules suivantes :
CH₂=CH-O(CH₂)ₐO(C₃H₆O)_{b}(C₂H₄O)_{c}H
ou
CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

12. Composition selon l'une quelconque des revendications précédentes, **caractérisée en ce que** le ou les monomères tensioactifs vinyliques semi-hydrophobes représentent de 0 à 25 % en poids, de préférence de 0,1 à 10 % en poids du mélange de monomères.

13. Composition selon l'une quelconque des revendications précédentes, **caractérisée en ce que** le ou les monomères vinyliques non ioniques hydroxylés d) sont choisis parmi les (méth)acrylates d'hydroxyalkyle en C₁-C₆, les (hydroxyalkyl en C₁-C₄)(méth)acrylamides, et leurs mélanges.

14. Composition selon la revendication 13, **caractérisée en ce que** le monomère vinylique non ionique hydroxylé est le méthacrylate de 2-hydroxyéthyle.

15. Composition selon l'une quelconque des revendications précédentes, **caractérisée en ce que** le ou les monomères vinyliques non ioniques hydroxylés représentent de préférence de 0,01 à 10 % en poids du mélange de monomères.

16. Composition selon la revendication précédente **caractérisée en ce que** le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10% en poids du ou des agents de transfert de chaîne,
h) de 0 à 2 % en poids du ou des stabilisateurs polymériques.

17. Composition selon l'une quelconque des revendications précédentes **caractérisée en ce que** ledit mélange de monomères comprend :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

18. Composition selon l'une quelconque des revendications précédentes, **caractérisée en ce que** le ou les polymères cationiques (i) sont présents à une concentration allant de 0,01 à 10% en poids par rapport au poids total de la composition, de préférence de 0,05 à 5% en poids et mieux de 0,1 à 1% en poids.

19. Composition selon l'une quelconque des revendications, **caractérisée par le fait que** la ou les cyclodextrines sont choisies parmi les α-cyclodextrines, les β-cyclodextrines ou les γ-cyclodextrines et leurs dérivés méthylés.

20. Composition selon la revendication 19, **caractérisée par le fait que** la ou les cyclodextrines sont choisies parmi les β-cyclodextrines.

21. Composition selon l'une quelconque des revendications 1 à 20, **caractérisée en ce que** la ou les cyclodextrines représentent de 0,2% à 30 % en poids, de préférence de 0,5 % à 15 % en poids, par rapport au poids total de la composition finale.

22. Composition selon l'une quelconque des revendications 1 à 4, **caractérisée en ce que** le ou les tensioactifs et la ou les cyclodextrines sont présents dans des concentrations efficaces pour former un complexe insoluble dans la composition, et/ou pour nacrer la composition.

23. Composition selon l'une quelconque des revendications précédentes, **caractérisée en ce que** le ou les tensioactifs sont anioniques.

24. Composition selon l'une quelconque des revendications 1 à 23, **caractérisée en ce que** le ou les tensioactifs est (sont) présent(s) dans des concentrations allant de 0,2% à 40% en poids, de préférence de 1% à 35% par rapport au poids total de la composition.

25. Composition selon l'une quelconque des revendications précédentes, **caractérisée en ce qu'**elle comprend en outre un ou plusieurs agents de conditionnement.

26. Procédé de traitement cosmétique des matières kératiniques en particulier des cheveux, **caractérisé par le fait qu'**il consiste à appliquer sur lesdites matières une composition telle que définie selon l'une quelconque des revendications 1 à 25 puis à effectuer éventuellement un rinçage à l'eau.
